Europäisches Patentamt

European Patent Office

Office européen des brevets

(19)

(11) Publication number: 0 298 414
A2

# (12) EUROPEAN PATENT APPLICATION

(21) Application number: 88110685.0

(51) Int. Cl.⁴: C12N 9/52

(22) Date of filing: 05.07.88

(30) Priority: 08.07.87 JP 170750/87

(43) Date of publication of application:
11.01.89 Bulletin 89/02

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Kuramoto, Tateyuki
816-8, Oaza-mitsui
Hikari Yamaguchi 743(JP)
Inventor: Agawa, Tamio
2047 Aza-hayashi Oaza-shimata
Hikari Yamaguchi 743(JP)
Inventor: Kishimoto, Katsumitsu
3784-3, Oaza-murozumison
Hikari Yamaguchi 743(JP)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) Method for purification of acid protease.

(57) The present invention concerns a method of purifying acid protease which comprises contacting a substantially salt-free aqueous solution of acid protease with a weakly basic ion exchange resin by use of substantially salt-free water and eluting the thus-adsorbed acid protease with an aqueous solution containing an inorganic salt.

The purification method of the present invention assures a high degree of purification without use of a concentrated salt solution or an organic solvent and can be used with advantage for commercial purification of acid protease.

EP 0 298 414 A2

EP 0 298 414 A2

## Method for Purification of Acid Protease

The present invention relates to a method of purifying acid protease which is useful as, for example, an antiinflammatory agent.

Serrapeptase (generic name) is a characteristic zinc metal-bearing acid protease produced by certain microorganisms and on the strength of its strong antiinflammatory activity has been used widely as, for example, an antiinflammatory drug.

Serrapeptase is generally manufactured by the steps of culture, separation of cells, and purification.

In the conventional method for separating bacterial cells from a culture broth of a serrapeptase-producing organism, a salt such as sodium sulfate, ammonium sulfate or the like is added to the culture broth to flocculate the cells and, then, the resulting flocs are removed by means of a centrifugal machine or by pressure filtration with the aid of a filter aid such as diatomaceous earth.

For the purification of serrapeptase, in consideration of the instability of this enzyme and for the maintenance of its antiinflammatory activity, a salting-out procedure or a fractional precipitation procedure using a water-soluble organic solvent, or both, has been generally employed (U. S. Patent No. 3,691,014).

In a recently reported purification method, serrapeptase is first adsorbed on a polyacrylate non-ionic adsorbent resin in the presence of a salt and, then, desorbed (eluted) with a water-containing organic solvent such as aqueous methanol (Japanese Unexamined Patent Application Kokai No. 61-215330).

The hitherto-known purification methods for acid protease, such as serrapeptase, invariably require large quantities of materials such as salts, organic solvents, etc. and, also, large amounts of labor and cost, for example in the recovery of the organic solvent used. Furthermore, much labor and time are required for the collection of fine precipitates and, in column-wise operations, a sufficiently high flow rate cannot be obtained. Because of these disadvantages, none of the methods so far proposed are fully satisfactory for the commercial scale purification of acid protease which is unstable by nature.

It should also be noted that the specific activities (purities) of the serrapeptase products obtainable by these prior art methods are not sufficiently high.

The present inventors found that acid protease can be efficiently adsorbed on a weakly basic ion exchange resin even in the absence of a salt and can be desorbed without using an organic solvent.

The inventors further established a method in which the cells can be separated from the culture broth without using a salt to give a substantially salt-free aqueous solution of acid protease and found that the solution thus obtained can be directly subjected to purification.

Thus, the present invention provides a method of purifying acid protease which comprises contacting a substantially salt-free aqueous solution of acid protease with a weakly basic ion exchange resin by use of substantially salt-free water and eluting the thus-adsorbed acid protease with an aqueous solution containing an inorganic salt.

Among the acid protease, serrapeptase may be preferably mentioned. An aqueous solution containing serrapeptase can be obtained by cultivating a serrapeptase-producing strain of the genus Serratia in a culture medium and separating the bacterial cells from the resulting culture broth (U.S. Patent No. 3,691,014).

As an example of said serrapeptase-producing strain of the genus Serratia, there may be mentioned Serratia sp. $E_{15}$. This Serratia sp. $E_{15}$ strain was deposited with American Type Culture Collection (ATCC) as of May 15, 1967 under the accession number of ATCC 21074 and has been listed on its Catalog of Strains I, 15th Ed., 1982.

The substantially salt-free aqueous solution of acid protease, e.g. serrapeptase, can be directly obtained by subjecting a culture broth of such a strain to a membrane separatory procedure such microfiltration (MF), ultrafiltration (UF) or ceramic filtration (CF). Particularly, the application of MF (pore size: about 0.05-10 μm) enables one to directly obtain a cell-free, substantially salt-free aqueous solution of serrapeptase without resort to addition of a salt.

The term "substantially salt-free" is used herein to mean an aqueous solution containing only the salts initially present in the culture broth, the usual salt concentration of which is not over 0.1%. Particularly preferred, from the standpoint of the efficiency of adsorption on a weakly basic ion exchange resin, is a solution whose salt concentration is not higher than 0.05% (w/v).

The serrapeptase content of such an aqueous solution is virtually optional but is usually in the range of 0.01 to 10% (w/v). The pH of the aqueous solution is preferably within the range of pH about 4-9 where serrapeptase remains stable. The substantially salt-free water for elution is preferably tap water.

The weakly basic ion exchange resin (OH-type) is an ion exchange resin based on a vinyl polymer, styrene polymer, acrylic acid polymer, DEAE-cellulose or the like and carrying weakly basic groups such as

2

primary, secondary or tertiary lower ($C_{1-4}$) alkylamino groups or the corresponding ammonium salts. In the practice of the present invention, an ion exchange resin based on a vinyl polymer and carrying weakly basic groups such as diethylamino, diethanolamino or the like can be employed with particular advantage.

The ion exchange resin is preferably used in the form of beads from 0.3 to 3.0 mm in diameter.

As examples of such weakly basic ion exchange resin, there may be mentioned EP-DA 13 L (Mitsubishi Chemical Industries, Ltd.,Japan) and Spezym GDC (Kurita Water Industries, Ltd., Japan).

The adsorption in the method of the invention is carried out in the per se conventional manner, e.g. columnwise or batchwise. This adsorption procedure is conducted at a temperature of 0-30°C and preferably at 0-10°C.

For adsorption, an aqueous solution containing serrapeptase is passed through a column of said resin at an SV of, for example, about 0.05-20 (a flow rate equal to about 0.05-20 times the volume of resin/hour) or, alternatively, the adsorbent resin is put into said aqueous solution and stirred to cause batchwise adsorption of serrapeptase. The elution is carried out by treating the resin carrying the serrapeptase adsorbed thereon with, for example, an aqueous solution containing an inorganic salt.

The inorganic salt mentioned above is preferably a neutral salt, and the salt of a strong acid with a strong base, such as sodium chloride, potassium chloride, sodium sulfate, sodium phosphate, ammonium sulfate, etc., can be used with particular advantage.

The inorganic salt-containing aqueous solution to be used for the elution of serrapeptase may be of low concentration, for example in the range of 1-10% (w/v) and preferably 4-6% (w/v).

The elution procedure is carried out at a temperature of about 0-30°C and preferably at 0-10°C.

As in the case of adsorption, this elution procedure may be columnwise or batchwise.

Some outstanding advantages of the acid protease purification method of the invention are as follows. The method assures a large adsorption capacity even without addition of a salt, a high elution yield even without use of an organic solvent, direct utilization of an impurity-rich crude enzyme solution, a high fractionating power leading to a marked improvement in specific enzyme activity, a high flow rate in columnwise operation on a commercial scale, and remarkable savings in labor because of the simplicity of adsorption and desorbtion procedures. Therefore, the method of the invention can be used advantageously as a purification step in the commercial production of acid protease.

## Examples

The following experimental and working examples are intended to illustrate the invention in further detail. The procedures described below were all carried out at about 10°C.

The enzyme potency unit Pu was determined by the method described in U. S. Patent No. 3,691,014.

## Experimental Example 1

An aqueous solution containing serrapeptase (serrapeptase activity 20,000 Pu/ml) was passed through a column of 100 ml of weakly basic ion exchange resin (OH-type) at a flow rate of 100 ml/hr to adsorb the serrapeptase. The relation between salt content and adsorption capacity was as shown in Table 1.

3

Table 1

| Salt content vs. adsorption capacity | | |
|---|---|---|
| Resin | Salt concentration of serrapeptase-containing aqueous solution | Adsorption of serrapeptase |
| Weakly basic ion exchange resin, FP-DA 13L | NaCl, 0.1% | 1 g/ℓ resin |
| Weakly basic ion exchange resin, FP-DA 13L | NaCl, 0.01% | 2 g/ℓ resin |
| Weakly basic ion exchange resin, FP-DA 13L | NaCl, 0.005% | 40 g/ℓ resin |
| Weakly basic ion exchange resin, FP-DA 13L | NaCl, 0% | 220 g/ℓ resin |

(1 mg is assumed to be 2,000 Pu)

It is clear that while serrapeptase is scarcely adsorbed in the presence of a salt, it is adsorbed with high efficiency when its aqueous solution is contacted with a weakly basic ion exchange resin in the presence of only a very small amount of a salt or in the absence of a salt.

## Experimental Example 2

An aqueous solution containing 6,000 Pu/ml of serrapeptase (1,000 ml) was passed through a column of 100 ml of FP-DA 13L to adsorb the serrapeptase. Then, elution was carried out with an aqueous solution containing a varying concentration of NaCl, i.e. 0%, 1%, 2%, 4% or 6%, to investigate the elution yields of serrapeptase.

Table 2

| Elution yields of serrapeptase | |
|---|---|
| NaCl concentration | Elution yield |
| 0% | 0 |
| 1% | 50 |
| 2% | 60 |
| 4% | 99 |
| 6% | 99 |

It is clear from Table 2 that elution could be achieved with high efficiencies using an aqueous solution of sodium chloride in the concentration range of 4 to 6%.

## Example 1

Serratia sp. E$_{15}$, a serrapeptase producer, was cultivated by the method described in Agricultural Biological Chemistry, 34, 310-318 (1970). The cells were removed from the resulting culture broth using an MF membrane (Microza, 0.1 μ, Asahi Kasei) to give a filtrate (serrapeptase activity 6,000 Pu/ml). Its salt content was 0.05 g/l and had a pH value of 6.9.

This filtrate, 3 liters, was passed through a column of 200 ml of weakly basic ion exchange resin (FP-DA 13L) at a rate of 200 ml/hr. After this adsorption of serrapeptase, the column was washed with tap water and, then, a 4% aqueous solution of NaCl was passed at a flow rate of 400 ml/hr. to desorb the serrapeptase.

The active fractions were pooled to give 400 ml of a purified serrapeptase solution (serrapeptase activity 44,500 Pu/ml). The specific activity of this solution was 10-fold as high as that of the MF filtrate.

## Example 2

The same solution as described in Example 1, 3 liters, was passed through a column of 400 ml of Spezym-GDC at a flow rate of 800 ml/hr. After this adsorption of serrapeptase, the column was washed with 400 ml of tap water and, then, elution was carried with a 4% aqueous solution of $(NH_4)_2SO_4$ at a flow rate of 800 ml/hr to desorb the serrapeptase.

The active fractions were pooled to give 800 ml of a filtrate. This filtrate was further subjected to fractional concentration using a ultrafiltration membrane to give a concentrated serrapeptase solution. The specific activity of this solution was 17-fold as high as that of the MF filtrate.

## Claims

1. A method of purifying acid protease which comprises contacting a substantially salt-free aqueous solution of acid protease with a weakly basic ion exchange resin by use of substantially salt-free water and eluting the thus-adsorbed acid protease with an aqueous solution containing an inorganic salt.

2. The method according to claim 1, wherein the acid protease is serrapeptase.

3. The method according to claim 1, wherein the aqueous solution of acid protease is obtained by cultivating a serrapeptase-producing strain of the genus Serratia in a culture medium and separating the bacterial cell from the resulting culture broth.

4. The method according to claim 3, wherein the separation of the bacterial cell is conducted by microfiltration, ultrafiltration or ceramic filtration.

5. The method according to claim 1, wherein the aqueous solution of acid protease contains serrapeptase in a concentration of 0.01 to 10% (w/v).

6. The method according to claim 1, wherein the aqueous solution of acid protease contains salts in a concentration of not more than 0.1% (w/v).

7. The method according to claim 1, wherein the weakly basic ion exchange resin is an ion exchange resin based on a vinyl polymer, styrene polymer, acrylic acid polymer or DEAE-cellulose which carries weakly basic groups.

8. The method according to claim 7, wherein the weakly basic ion exchange is used in the form of beads from 0.3 to 3.0 mm in diameter.

9. The method according to claim 1, wherein the substantially salt-free water contains salt in a concentration not higher than 0.01% (w/v).

10. The method according to claim 1, wherein the aqueous solution contains an inorganic salt in a concentration of from 1 to 10% (w/v) and is free from an organic solvent.

11. The method according to claim 1, wherein the absorption process is carried out by columnwise or batchwise.

12. The method according to claim 11, wherein the columnwise process is conducted by SV of 0.05 to 20 of the flow rate.

13. A method of purifying acid protease which comprises cultivating a acid protease-producing strain in a culture medium, separating the bacterial cell from the culture broth, contacting the resulting substantially salt-free aqueous solution of acid protease with a weakly basic ion exchange resin by use of substantially salt-free water and eluting the thus-adsorbed acid protease with an aqueous solution containing an inorganic salt.